(19) Europäisches Patentamt
European Patent Office
Office européen des brevets

(11) Veröffentlichungsnummer: **0 034 727**
**B1**

# (12) EUROPÄISCHE PATENTSCHRIFT

(45) Veröffentlichungstag der Patentschrift: **14.11.84**

(21) Anmeldenummer: **81100674.1**

(22) Anmeldetag: **30.01.81**

(51) Int. Cl.³: **C 01 B 33/28**, C 01 B 33/20, C 01 B 35/12, C 01 G 17/00, C 01 G 49/00, B 01 J 29/04, C 07 C 1/20

## (54) Kristalline isotaktische Zeolithe, Verfahren zur Herstellung derselben sowie deren Verwendung als Katalysatoren.

(30) Priorität: **21.02.80 DE 3006471**

(43) Veröffentlichungstag der Anmeldung: **02.09.81 Patentblatt 81/35**

(45) Bekanntmachung des Hinweises auf die Patenterteilung: **14.11.84 Patentblatt 84/46**

(84) Benannte Vertragsstaaten: **BE DE FR GB NL**

(56) Entgegenhaltungen: **EP - A - 0 007 081**

(73) Patentinhaber: **BASF Aktiengesellschaft, Carl-Bosch-Strasse 38, D-6700 Ludwigshafen (DE)**

(72) Erfinder: **Marosi, Laszlo, Dr., Leuschner-Strasse 32, D-6700 Ludwigshafen (DE)**
Erfinder: **Stabenow, Joachim, Dr., Am Feldrain 22, D-6940 Weinheim (DE)**
Erfinder: **Schwarzmann, Matthias, Dr., Carl-Bosch-Strasse 54, D-6703 Limburgerhof (DE)**

**EP 0 034 727 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neuartige Zeolithe, ein Verfahren zu ihrer Herstellung, sowie die Anwendung dieser Zeolithe als Katalysatoren.

Zeolithe des A-, X-, Y- und Mordenit-Typs haben eine grosse technische Bedeutung erlangt. Sie werden als Ionenaustauscher, Molekularsiebe und Katalysatoren technisch eingesetzt. Technische Verfahren, wie das Katalytische und hydrierende Cracken von Kohlenwasserstoffen werden mit Zeolithkatalisatoren durchgeführt. Neuerdings gewinnen Zeolithe vom Typ ZSM-5 zunehmend an Interesse, z.B. für Reaktionen wie die Umwandlung von Methanol in Aromaten und/oder Olefine.

Zeolithe sind kristalline Silikate, insbesondere Aluminosilikate. Zu ihrer Herstellung werden reaktive $SiO_2$- und $Al_2O_3$-Ausgangsmaterialien in Gegenwart von starken Basen hydrothermal zur Kristallisation gebracht. Als starke Base werden Alkali- oder Erdalkalihydroxide oder quaternäre Stickstoff- oder Phosphorbasen, entweder allein oder in Mischungen miteinander verwendet. Ein Nachteil dieser Synthesemethode besteht in der Verwendung von schwer zugänglichen quaternären Stickstoffbasen oder darin, dass die Alkalikationen gegen andere Kationen ausgetauscht werden müssen, damit die katalytisch aktive Form entsteht. Eine gewisse Vereinfachung bedeutet die Verwendung von primären Aminen oder Diaminen in Verbindung mit Alkaliionen an Stelle der quaternären Stickstoffbasen bei der Herstellung von ZSM-5 artigen Zeolithen. Diese bekannten Verfahren sind z.B. in den DE-Offenlegungsschriften 2 442 240, 2 817 575 und 2 817 576 beschrieben. Ein gemeinsames Merkmal dieser Herstellungsverfahren besteht darin, dass die Synthese stets in Gegenwart von Alkaliionen erfolgt und auch die entstehenden Zeolithe daher alkalihaltig sind. Andere Verfahren, die in Abwesenheit von Alkaliionen durchgeführt werden, verwenden die als starke Basen geltenden quaternären Stickstoffbasen zur Kristallisation.

Gegenstand der Erfindung ist ein Verfahren zur Herstellung von kristallinen isotaktische Zeolithen der Zusammensetzung

$$2/_nR \cdot W_2O_3 \cdot m\ YO_2 \cdot p\ H_2O$$

worin R ein organisches Amin und n die Zahl der Amingruppen in dem Aminmolekül bedeutet, W die Elemente B, Al, Ga und Fe, Y die Elemente Si und Ge einschliesst und m den Wert von

$$m = (1 \pm 0{,}15)\,\frac{96 - \dfrac{40\,n}{L_R + K}}{\dfrac{20\,n}{L_R + K}}$$

besitzt, wobei $L_R$ die Länge des Aminmoleküls in Å bedeutet und K eine Konstante ist deren Wert etwa 4 beträgt, und p eine Zahl von 0 bis 160 ist und das kristalline isotaktische Zeolithmaterial mindestens folgende Beugungslinien aufweist:

$$d(Å) = 11{,}1 \pm 0{,}3/10{,}0 \pm 0{,}25/3{,}85 \pm 0{,}1/\ 3{,}71 \pm 0{,}1/3{,}54 \pm 0{,}07$$

aus Mischung von $YO_2$ und $W_2O_3$ durch hydrothermaler Kristallisation bei Temperaturen von 100 bis 200 °C, wobei man die Kristallisation in Abwesenheit von Alkali-, Erdalkali- oder quaternären Stickstoffbasen oder deren Vorläufer mit Hilfe von organischen Aminen durchführt und dabei in der Reaktionsmischung in Abhängigkeit von Kettenlänge $L_R$ des organischen Amins ein zur Bildung des isotaktischen Zeoliths geeignetes Molverhältnis von $YO_2$ zu $W_2O_3$ einstellt, wobei die molaren Verhältnisse von $H_2O$/Amin im Bereich von 0,4 bis 100 und von Amin/$Al_2O_3$ im Bereich von 5 bis 400 liegen.

Zweckmässig werden Mischungen von $W_2O_3$ und $YO_2$ in Gegenwart eines organischen Amins bei Temperaturen zwischen 100 bis 200 °C 1 bis 15 Tage hydrothermal behandelt.

Vorteilhaft gibt man dabei nur soviel $W_2O_3$ zu, als zur Bildung des kristallinen, isotaktischen Zeoliths erforderlich ist. Verwendet man $Al_2O_3$, so entspricht die Zugabe der berechneten Menge. Borsäure ist im Gegensatz zu $Al(OH)_3$ in Wasser löslich. Deshalb wird zweckmässig in der Reaktionsmischung ein kleineres $SiO_2/B_2O_3$-Verhältnis verwendet als der Zusammensetzung des kristallinen Zeoliths entspricht.

Erfindungsgemäss erhält man Kristalline isotaktische Zeolithe der Zusammensetzung

$$2/_nR \cdot W_2O_3 \cdot m\ YO_2 \cdot p\ H_2O$$

worin R Dipropylentriamin, Dihexamethylentriamin, Triäthylentetramin, Propylendiamin und Diäthylentriamin und n die Zahl der Amingruppen in dem Aminmolekül bedeutet, W die Elemente B, Al, Ga und Fe, Y die Elemente Si und Ge einschliesst und m den Wert von

$$m = (1 \pm 0{,}15)\,\frac{96 - \dfrac{40\,n}{L_R + K}}{\dfrac{20\,n}{L_R + K}}$$

besitzt, wobei $L_R$ die Länge des Aminmoleküls in Å bedeutet und K eine Konstante ist deren Wert etwa 4 beträgt, und p eine Zahl von 0 bis 160 ist und das kristalline isotaktische Zeolithmaterial mindestens folgende Beugungslinien aufweist:

$$d(Å) = 11{,}1 \pm 0{,}3/10{,}0 \pm 0{,}25/3{,}85 \pm 0{,}1/\ 3{,}71 \pm 0{,}1/3{,}54 \pm 0{,}07$$

Es wurde weiter gefunden, dass die erfindungsgemässen Zeolithe mit besonderen Eigenschaften in Abwesenheit von starken Basen erhält, wenn man die hydrothermale Kristallisation mit Hilfe von wesentlich schwächer basischen organischen Aminen durchführt.

Die bei dem erfindungsgemässen Verfahren entstehenden Zeolithe weisen besondere Eigenschaften auf, die sie von anderen bekannten Zeolithen z.B. den mit Hilfe von Alkali oder quaternären Stickstoffbasen hergestellten, auch bei Vorliegen des gleichen Strukturtyps unterscheiden. Zeolithe mit diesen Eigenschaften sind weder durch Ionenaustausch noch durch andere Modifizierungen aus bekannten Zeolithtypen herstellbar, noch können sie durch bekannte Herstellungsverfahren durch Direktsynthese erzeugt werden. Die neuartigen Eigenschaften sind bedingt durch die erfindungsgemässe Zusammensetzung, die man durch die besondere Zusammensetzung der Reaktionsmischung erhält, die auf die Kettenlänge des zur Kristallisation verwendeten Amins abgestimmt ist.

Das erfindungsgemässe Verfahren wird in Gegenwart von organischen Aminen, die eine wesentlich schwächere Basizität als Alkali oder quaternäre Stickstoffbasen aufweisen, z.B. primären oder sekundären Aminen, wie Dipropylentriamin, Dihexamethylentriamin, Hexamethylendiamin, Propylendiamin, Diäthylentriamin oder Triäthylentriamin oder in Gegenwart von Mischungen dieser Amine durchgeführt.

Die besonderen Eigenschaften dieser neuartigen Zeolithmaterialien sind zurückzuführen auf eine geordnete (isotaktische) Anordnung der 3-wertigen Metallatome, z.B. Aluminiumatome im Gitter, die von den herkömmlichen Zeolithen des gleichen Strukturtyps, die in Gegenwart von Alkali oder quaternären Stickstoff- oder Phosphorbasen hergestellt werden, abweicht. Die neuen Zeolithe weisen eine charakteristische untere Grenze des $SiO_2/W_2O_3$-Molverhältnisses auf, das von der Kettenlänge und Zahl der Aminogruppen des verwendeten Amins abhängig ist.

Wenn man z.B. $SiO_2$-$Al(OH)_3$-Mischungen in Gegenwart von Hexamethylendiamin unter den oben genannten Bedingungen kristallisiert, entstehen Zeolithe, die im folgenden mit ZBM-10 bezeichnet werden, und die eine den bekannten Zeolithen ZSM-5, ZSM-8 und ZSM-11 ähnliche Struktur aufweisen, von diesen aber mit Hilfe der Röntgenbeugungsanalyse eindeutig zu unterscheiden sind.

Insbesondere wurde gefunden, dass z.B. die $SiO_2$-$Al(OH)_3$-Mischungen mit Hilfe von Hexamethylendiamin nur dann zur Kristallisation gebracht werden können, wenn das $SiO_2/Al_2O_3$-Molverhältnis mindestens etwa 33 beträgt. Wenn man $SiO_2$-$Al(OH)_3$-Mischungen mit einem $SiO_2/Al_2O_3$-Molverhältnis von etwa 10 bis 20 mit Hilfe von Hexamethylendiamin bei Temperaturen zwischen 150 und 190 °C in Abwesenheit von Alkali oder quaternären Stickstoffbasen kristallisieren will, erhält man dagegen im wesentlichen amorphe Produkte. Solche Mischungen ergeben nur dann kristalline Zeolithe, wenn die Reaktionsmischung starke Basen enthält.

Dieses Ergebnis ist überraschend, da es bekannt ist, dass nach den herkömmlichen Verfahren Zeolithe vom ZSM-5-Typ mit $SiO_2/Al_2O_3$-Molverhältnissen ab etwa 5 herstellbar sind, auch wenn die Kristallisation mit Hilfe von Hexamethylendiamin, in Gegenwart von Alkali, durchgeführt wird.

Der Zusammenhang zwischen Kettenlänge des Amins, Zahl der Amingruppen im Aminmolekül, und maximalem Aluminiumgehalt im Zeolithkristall gilt auch bei Verwendung anderer schwach basischer Amine. Die $YO_2/W_2O_3$-Verhältnisse variieren dabei in charakteristischer Weise gemäss der oben genannten Formel.

Die nachfolgende Tabelle 1 gibt den gesetzmässigen Zusammenhang zwischen der Kettenlänge des verwendeten Amins und dem maximalen Gehalt an Aluminium im Kristallgitter als Ergebnis der Kristallisationsversuche wieder.

Tabelle 1

| R | $L_R$ (Å) | $96 - \dfrac{96 - \dfrac{40\,n}{L_R+4}}{\dfrac{20\,n}{L_R+4}}$ | $SiO_2/Al_2O_3$-Molverhältnis | Produkt |
|---|---|---|---|---|
| Dipropylentriamin | 12,0 | 23,6 | 23 | amorph |
| | | | 26 | kristallin |
| Hexamethylendiamin | 10,7 | 33,3 | 30 | amorph |
| | | | 33 | kristallin |
| | | | 35 | kristallin |
| Dihexamethylentriamin | 20,0 | 36,4 | 33 | amorph |
| | | | 38 | kristallin |
| Triäthylentetramin | 13,4 | 18,9 | 19 | kristallin |
| | | | 15 | amorph |
| Propylendiamin | 6,7 | 23,7 | 22 | kristallin |
| Diäthylentriamin | 9,4 | 19,4 | 20 | kristallin |
| | | | 15 | amorph |

Die Tabelle belegt experimentell den Zusammenhang der besonderen Eigenschaften dieser Zeolithe mit dem besonderen strukturellen Aufbau. Der Abstand zweier z.B. Aluminiumatome, im Kristallgerüst des Zeoliths ist durch den Abstand der Aminogruppen des Aminmoleküls gegeben und wiederholt sich in periodischen Abständen.

Die erfindungsgemässen Zeolithe, in Gegenwart von verschiedenen Aminen hergestellt, wurden auf ihre katalytische Aktivität u.a. für die Methanolzersetzung zu Kohlenwasserstoffen geprüft.

Es wurde gefunden, dass der Zeolith ZBM-10 ($Si/Al_2 = 33$) bei der Umsetzung von Methanol-Wasser-Gemischen im molaren Verhältnis 1 : 5

überwiegend Olefine ergibt, während ZBM-11, ein mit Hilfe von Borsäure anstelle von $Al(OH)_3$ und Hexamethylendiamin und ZBM-12 ein mit Hilfe von Aluminiumhydroxid und Dipropylentriamin hergestellter Zeolith bei dem gleichen Verhältnis $SiO_2/B_2O_3$ bzw. $SiO_2/Al_2O_3$ konzentrierte Methanollösungen im Bereich von 85 bis 100% in überwiegend Propylen (ZBM-11) oder in überwiegend Äthylen und Propylen (ZBM-12) umsetzt. Unter den gleichen Reaktionsbedingungen ergab ZBM-10-Zeolith mit 95%igem Methanol überwiegend flüssige Kohlenwasserstoffe.

Diese Ergebnisse beweisen, dass es möglich ist, Zeolithe vom Typ Aluminosilikat, Borosilikat, Eisensilikat oder Mischkristallen von diesen herzustellen, die Methanol unter sonst gleichen Reaktionsbedingungen ohne jede weitere Massnahme, je nach verwendetem Katalysator, zu verschiedenen Produkten umsetzen, z.B. zu einem Gemisch aus Äthylen und Propylen oder einem Gemisch das überwiegend Propylen enthält oder einem Gemisch aus flüssigen Kohlenwasserstoffen.

Die Verwendung der erfindungsgemässen Zeolithe als Katalysatoren bietet gegenüber den bekannten Zeolithen grosse verfahrenstechnische Vorteile:

a) Reines Methanol oder Rohmethanol können ohne weitere Massnahme in Olefine umgewandelt werden, wobei die Belastung bei 100% Umsatz 10 g Methanol/g Zeolith betragen kann und
b) die Katalysatoren z.B. Zeolith ZBM-11, geringere Verkohlungsraten aufweisen als vergleichbare bekannte Zeolithe.

Die erfindungsgemässen Zeolithe unterscheiden sich nicht nur durch ihre besondere Zusammensetzung, die durch ihre besondere Herstellung bedingt ist, von anderen bekannten Typen der ZSM-5-Familie.

Der Zeolith ZBM-11 unterscheidet sich z.B. dadurch von anderen ZSM-5-Typen, dass er alkalifrei und/oder frei von quaternären Stickstoffbasen ist und sein Röntgenbeugungsdiagramm eindeutig von den Beugungsdiagrammen anderer bekannter Metallsilikate ähnlichen Strukturtyps unterscheidbar ist. Besonders charakteristisch ist dabei die Verschiebung der Röntgenbeugungslinien nach kleineren d-Werten wie beispielsweise bei X- und Y-Zeolithen oder der davon ableitbaren $SiO_2$-Modifikation mit Faujasitstruktur und beispielsweise das Fehlen der relativ intensiven Beugungslinien bei d = 4,27 Å, wenn man HMD zur Kristallisation verwendet.

Die erfindungsgemässen Zeolithe können z.B. hergestellt werden, indem man Mischungen von reaktivem $SiO_2$, wie pyrogene Kieselsäure (Aerosil) und aktivem Aluminiumoxid, beispielsweise frisch gefälltem $Al(OH)_3$ oder $B(OH)_3$ oder $Fe(OH)_3$ in Gegenwart von organischen Aminen unter hydrothermalen Bedingungen kristallisiert. Als Kristallisationsmittel kommen, ausser den quaternären Stickstoffbasen oder deren Vorläufer, alle Amine in Frage, deren Kettenlänge mindestens etwa 6 Å beträgt.

Eine besondere Ausführungsform zur Herstellung der neuen Na-freien Zeolithe besteht darin, dass man z.B. eine Reaktionsmischung aus $SiO_2$ und Aluminiumoxid bzw. -hydroxid oder deren natriumfreie Vorläufer in dem oben angegebenen Verhältnis mischt und dann in einer wässrigen Aminlösung 2 Stunden bis 8 Tage auf Temperaturen zwischen 100 und 200 °C, vorzugsweise 1 bis 4 Tage auf 140 bis 170 °C unter ihrem Eigendruck erhitzt. Die so hergestellten Zeolithe enthalten in der Regel grössere Mengen des verwendeten Amins in den intrakristallinen Poren eingelagert. Dieses Amin kann beispielsweise durch Verbrennen aus den Poren entfernt werden, wobei die katalytisch aktive Wasserstofform gebildet wird.

Es ist vorteilhaft, dass die Mutterlauge in vollem Umfang wieder zur Herstellung von neuem Zeolith eingesetzt werden kann.

Beispiel 1

In 1200 g 50%ige Dipropylentriaminlösung werden 101 g Aerosil und frisch gefälltes $Al(OH)_3$ eingetragen. Das $Al(OH)_3$ wird aus 49 g $Al(NO_3)_3 \cdot 9\ H_2O$ durch Fällen mit Ammoniak hergestellt. Die Mischung wird dann so lange gerührt, bis sie homogen ist und anschliessend 7 Tage in einem Stahlautoklav auf 170 °C erhitzt. Das kristalline Produkt wird filtriert, gewaschen und bei 100 °C getrocknet. Laut Röntgenanalyse besteht es aus gut kristallisiertem Aluminiumzeolith vom Pentasil-Typ.

Beispiel 2

Es werden Mischungen von $SiO_2$ und $Al(OH)_3$ hergestellt und in der im Beispiel 1 beschriebenen Weise 101 g $SiO_2$ und dem jeweiligen $SiO_2/Al_2O_3$-Molverhältnis entsprechende Menge $Al(OH)_3$ in 1200 g 50%iger Aminlösung 7 Tage bei 170 °C hydrothermal behandelt. Die Zusammensetzung der Reaktionsmischung, Kristallisationsbedingungen und das Ergebnis der Versuche sind in der Tabelle 2 zusammengestellt:

| R | Molverhältnis $SiO_2/Al_2O_3$ | Produkt |
|---|---|---|
| Dipropylentriamin | 23 | amorph |
| | 26 | kristallin |
| Hexamethylendiamin | 30 | amorph |
| | 33 | kristallin |
| | 35 | kristallin |
| Dihexamethylentriamin | 33 | amorph |
| | 38 | kristallin |
| Propylendiamin | 22 | kristallin |
| Triethylentetramin | 19 | kristallin |
| | 15 | amorph |
| Diäthylentriamin | 20 | kristallin |
| | 15 | amorph |

Beispiel 3

Es werden zwei Mischungen aus $SiO_2$ und $B(OH)_3$ hergestellt und in einer 50%igen Hexamethylendiaminlösung 5 Tage bei 150 °C hydrothermal behandelt.

In 2000 g 50%ige Hexamethylendiaminlösung werden 160 g Aerosil und in Versuch a) 40 g, in Versuch b) 30,4 g Borsäure eingetragen. Die Mischung wird dann jeweils so lange gerührt bis sie homogen ist und anschliessend 5 Tage in einem Stahlautoklav auf 150 °C erhitzt. Die Produkte werden filtriert, gewaschen und getrocknet. Laut Röntgenanalyse ist Produkt a) amorph. Das Produkt b) besteht aus gut kristallisiertem Borzeolith. Das $SiO_2/B_2O_3$-Verhältnis im Produkt b) beträgt 33. Der Versuch zeigt, dass ein maximaler Borgehalt von etwa $SiO_2/B_2O_3$ 33 den kristallinen Zeolith ergibt.

Beispiel 4

100 g des in Beispiel 3b) erhaltenen Zeoliths werden mit Böhmit zu 1-mm-Strängen verarbeitet. Der Zeolithgehalt der Stränge beträgt 65 Gew.%. 20 g des erhaltenen Produktes werden als Katalysator in einem Durchflussreaktor eingebaut und die Aktivität in verschiedenen Reaktionen getestet.

Herstellung von Olefinen aus Methanol

Rohmethanol mit einem Wassergehalt von 17 Gew.% wird bei 380 °C über den Katalysator geleitet. Die Belastung beträgt 10 g Rohmethanol/h und g Katalysator. Die Reaktionsbedingungen sind in der folgenden Tabelle zusammengefasst:

| | |
|---|---|
| Eingangstemperatur | 380 °C |
| Druck | 1,16 bar |
| Temperaturanstieg | 220 °C |
| Umsetzung | 100% |

Das erhaltene Reaktionsprodukt hat folgende Zusammensetzung: 22 Gew.% flüssige Kohlenwasserstoffe und 78 Gew.% gasförmige Reaktionsprodukte, bezogen auf eingesetztes $CH_2$. Die gasförmigen Produkte bestehen aus 5,6 Vol.% $CH_4$, 24 Vol.% $C_2H_4$, 3,7 Vol.% $C_3H_8$, 42 Vol.% $C_3H_6$, 5,6 Vol.% Butan und 20,3 Vol.% Butene.

Beispiel 5

20 g Dimethyläther/Stunde werden über 20 g eines nach Beispiel 3b erhaltenen Katalysator geleitet.

| | |
|---|---|
| Eingangstemperatur | 330 °C |
| Temperaturanstieg | 120 °C |
| Belastung | 1 g DMA/g Kat h. |

Man erhält 66 Gew.% flüssige Kohlenwasserstoffe, bestehend aus:

38 Gew.% Aromaten,
38,5 Gew.% Aliphaten und
23,5 Gew.% flüssigen Olefinen.

Beispiel 6

Es wird ein $C_4$-Schnitt bei Temperaturen von 360 bis 370 °C Eingangstemperatur bei einer Belastung von 2 g $C_4$-Schnitt/g Katalysator und Stunde über einem Katalysator umgesetzt, der nach Beispiel 3b erhalten worden ist.

Die Reaktionsprodukte bestehen aus 67 Gew.% flüssigen Kohlenwasserstoffen, die zu 75% aus Aromaten, 21% Aliphaten und 4% Olefinen bestehen.

**Patentansprüche**

1. Verfahren zur Herstellung von kristallinen isotaktischen Zeolithen der Zusammensetzung

$$2/_nR \cdot W_2O_3 \cdot m\,YO_2 \cdot p\,H_2O$$

worin R ein organisches Amin und n die Zahl der Amingruppen in dem Aminmolekül bedeutet, W die Elemente B, Al, Ga und Fe, Y die Elemente Si und Ge einschliesst und m den Wert von

$$m = (1 \pm 0{,}15)\,\frac{96 - \dfrac{40\,n}{L_R + K}}{\dfrac{20\,n}{L_R + K}}$$

besitzt, wobei $L_R$ die Länge des Aminmoleküls in Å bedeutet und K eine Konstante ist deren Wert etwa 4 beträgt, und p eine Zahl von 0 bis 160 ist und das kristalline isotaktische Zeolithmaterial mindestens folgende Beugungslinien aufweist:

$$d(Å) = 11{,}1 \pm 0{,}3/10{,}0 \pm 0{,}25/3{,}85 \pm 0{,}1/3{,}71 \pm 0{,}1/3{,}54 \pm 0{,}07$$

aus Mischungen von $YO_2$ und $W_2O_3$ durch hydrothermale Kristallisation bei Temperaturen von 100 bis 200 °C, dadurch gekennzeichnet, dass man die Kristallisation in Abwesenheit von Alkali-, Erdalkali- oder quaternären Stickstoffbasen oder deren Vorläufer mit Hilfe von organischen Aminen durchführt und dabei in der Reaktionsmischung in Abhängigkeit von der Kettenlänge $L_R$ des organischen Amins ein zur Bildung des isotaktischen Zeoliths geeignetes Molverhältnis von $YO_2$ zu $W_2O_3$ einstellt, wobei die molaren Verhältnisse von $H_2O$/Amin im Bereich von 0,4 bis 100 und von Amin/$Al_2O_3$ im Bereich von 5 bis 400 liegen.

2. Verfahren zur Herstellung von kristallinen isotaktischen Aluminosilikatzeolithen nach Anspruch 1 aus Mischungen von $SiO_2$ und $Al(OH)_3$ durch hydrothermale Kristallisation bei Temperaturen von 100 bis 200 °C, dadurch gekennzeichnet, dass man die Kristallisation in Abwesenheit von Alkali-, Erdalkali- oder quaternären Stickstoffbasen oder deren Vorläufer mit Hilfe von organischen Aminen durchführt und dabei in der Reaktionsmischung in Abhängigkeit von der Kettenlänge $L_R$ des organischen Amins ein Molverhältnis von

$$SiO_2/Al_2O_3 = (1 \pm 0{,}15)\,\frac{96 - \dfrac{40\,n}{L_R + 4}}{\dfrac{20\,n}{L_R + 4}}$$

verwendet und die molaren Verhältnisse von $H_2O$/Amin im Bereich von 0,4 bis 100 und von Amin/$Al_2O_3$ im Bereich von 5 bis 400 liegen.

3. Verfahren zur Herstellung von kristallinen isotaktischen Borosilikatzeolithen nach Anspruch 1 aus Mischungen von $SiO_2$ und $B(OH)_3$ durch hydrothermale Kristallisation bei Temperaturen von 100 bis 200 °C, dadurch gekennzeichnet, dass

man die Kristallisation in Abwesenheit von Alkali-, Erdalkali- oder quaternären Stickstoffbasen oder deren Vorläufer mit Hilfe von organischen Aminen durchführt und dabei in der Reaktionsmischung nur soviel $B(OH)_3$ zusetzt als zur Bildung des kristallinen, isotaktischen Borosilikatzeoliths erforderlich ist.

4. Verfahren nach Anspruch 1 bis 3, dadurch gekennzeichnet, dass man die hydrothermale Kristallisation mit Dipropylentriamin ausführt.

5. Verfahren nach Anspruch 1 bis 4, dadurch gekennzeichnet, dass man die hydrothermale Kristallisation mit Propylendiamin ausführt.

6. Verfahren nach Anspruch 1 bis 5, dadurch gekennzeichnet, dass man die hydrothermale Kristallisation mit Dihexamethylentriamin ausführt.

7. Verfahren nach Anspruch 1 bis 6, dadurch gekennzeichnet, dass man die hydrothermale Kristallisation mit Triethylentetramin ausführt.

8. Verfahren nach Anspruch 1 bis 7, dadurch gekennzeichnet, dass man die hydrothermale Kristallisation mit Diethylentriamin ausführt.

9. Verfahren zur Herstellung von kristallinen isotaktischen Zeolithen nach Anspruch 1 bis 18, dadurch gekennzeichnet, dass man durch Entfernen der Aminkomponente die H-Form der Zeolithe gewinnt.

10. Kristalline isotaktische Zeolithe der Zusammensetzung

$$2/_nR \cdot W_2O_3 \cdot m\ YO_2 \cdot p\ H_2O$$

worin R Dipropylentriamin, Dihexamethylentriamin, Triäthylentetramin, Propylendiamin und Diäthylentriamin und n die Zahl der Amingruppen in dem Aminmolekül bedeutet, W die Elemente B, Al, Ga und Fe, Y die Elemente Si und Ge einschliesst und m den Wert von

$$m = (1 \pm 0{,}15)\,\frac{96 - \dfrac{40\ n}{L_R + K}}{\dfrac{20\ n}{L_R + K}}$$

besitzt, wobei $L_R$ die Länge des Aminmoleküls in Å bedeutet und K eine Konstante ist, deren Wert etwa 4 beträgt, und p eine Zahl von 0 bis 160 ist und das kristalline isotaktische Zeolithmaterial mindestens folgende Beugungslinien aufweist:

$$d(Å) = 11{,}1 \pm 0{,}3/10{,}0 \pm 0{,}25/3{,}85 \pm 0{,}1/ \quad 3{,}71 \pm 0{,}1/3{,}54 \pm 0{,}07$$

11. Verwendung von Zeolithen gemäss Anspruch 10 als Katalysator für die Umwandlung von Alkoholen und/oder Dialkyläthern in Olefine und/oder Aromaten.

**Claims**

1. A process for the preparation of a crystalline isotactic zeolite of the composition

$$2/_nR \cdot W_2O_3 \cdot m\ YO_2 \cdot p\ H_2O$$

where R is an organic amine and n is the number of amine groups in the amine molecule, W comprises the elements B, Al, Ga and Fe, Y comprises the elements Si and Ge, and m is

$$m = (1 \pm 0{,}15)\,\frac{96 - \dfrac{40\ n}{L_R + K}}{\dfrac{20\ n}{L_R + K}}$$

where $L_R$ is the length of the amine molecule in Å, K is a constant having a value of about 4, p is a number from 0 to 160 and the crystalline isotactic zeolite material exhibits one or more of the following diffraction lines

$$d(Å) = 11.1 \pm 0.3/10.0 \pm 0.25/3.85 \pm 0.1 \quad 3.71 \pm 0.1/3.54 \pm 0.07,$$

from a mixture of $YO_2$ and $W_2O_3$ by hydrothermal crystallization at from 100 to 200 °C, wherein the crystallization is carried out in the absence of an alkali metal base, alkaline earth metal base or quaternary nitrogen base or their intermediates, with the aid of an organic amine, the molar ratio of $YO_2$ to $W_2O_3$ in the reaction mixture being brought to a value, depending on the chain length $L_R$ of the organic amine, which is appropriate for the formation of the isotactic zeolite, the molar ratio $H_2O$/amine being from 0.4 to 100 and the molar ratio amine/$Al_2O_3$ being from 5 to 400.

2. A process for the preparation of a crystalline isotactic aluminosilicate zeolite as claimed in claim 1 from a mixture of $SiO_2$ and $Al(OH)_3$, by hydrothermal crystallization at from 100 to 200 °C, wherein the crystallization is carried out in the absence of alkali metal bases, alkaline earth metal bases or quaternary nitrogen bases or their intermediates, with the aid of an organic amine, and using, in the reaction mixture, a molar ratio $SiO_2/Al_2O_3$ which depends on the chain length $L_R$ of the organic amine and is given by

$$SiO_2/Al_2O_3 = (1 \pm 0{,}15)\,\frac{96 - \dfrac{40\ n}{L_R + 4}}{\dfrac{20\ n}{L_R + 4}}$$

the molar ratio $H_2O$/amine being from 0.4 to 100 and the molar ratio amine/$Al_2O_3$ being from 5 to 400.

3. A process for the preparation of a crystalline isotactic borosilicate zeolite as claimed in claim 1 from a mixture of $SiO_2$ and $B(OH)_3$, by hydrothermal crystallization at from 100 to 200 °C, wherein the crystallization is carried out in the absence of alkali metal bases, alkaline earth metal bases or quaternary nitrogen bases or of their intermediates, with the aid of organic amines, and only as much $B(OH)_3$ is added to the reaction mixture as is required for the formation of the crystalline, isotactic borosilicate zeolite.

4. A process as claimed in claims 1 to 3, wherein the hydrothermal crystallization is carried out with dipropylenetriamine.

5. A process as claimed in claims 1 to 4, wherein the hydrothermal crystallization is carried out with propylenediamine.

6. A process as claimed in claims 1 to 5, wherein the hydrothermal crystallization is carried out with dihexamethylenetriamine.

7. A process as claimed in claims 1 to 6, wherein the hydrothermal crystallization is carried out with triethylenetetramine.

8. A process as claimed in claims 1 to 7, wherein the hydrothermal crystallization is carried out with diethylenetriamine.

9. A process for the preparation of a crystalline isotactic zeolite as claimed in claims 1 to 8, wherein the H form of the zeolite is obtained by removing the amine component.

10. A crystalline isotactic zeolite of the composition

$$2/_nR \cdot W_2O_3 \cdot m\ YO_2 \cdot p\ H_2O$$

where R is dipropylenetriamine, dihexamethylenetriamine, triethylenetetramine, propylenediamine or diethylenetriamine, and n is the number of amine groups in the amine molecule, W comprises the elements B, Al, Ga and Fe, Y comprises the elements Si and Ge, and m is

$$m = (1 \pm 0,15) \frac{96 - \frac{40\ n}{L_R + K}}{\frac{20\ n}{L_R + K}}$$

where $L_R$ is the length of the amine molecule in Å, K is a constant having a value of about 4, p is a number from 0 to 160 and the crystalline isotactic zeolite material exhibits one or more of the following diffraction lines

d(Å) = 11.1±0.3/10.0±0.25/3.85±0.1/
3.71±0.1/3.54±0.07.

11. The use of a zeolite as claimed in claim 10 as a catalyst for the conversion of alcohols and/or dialkyl ethers to olefins and/or aromatics.

**Revendications**

1. Procédé de préparation de zéolithes isotactiques cristallines de la composition

$$2/_nR \cdot W_2O_3 \cdot m\ YO_2 \cdot p\ H_2O,$$

dans laquelle R désigne une amine organique et n indique le nombre de groupes amine dans la molécule d'amine, W représente les éléments B, Al, Ga et Fe et Y les éléments Si et Ge et m possède la valeur

$$m = (1 \pm 0.15) \frac{96 - \frac{40\ n}{L_R + K}}{\frac{20\ n}{L_R + K}}$$

où $L_R$ est la longueur de la molécule d'amine en Å, K est une constante d'une valeur d'environ 4 et p est un nombre entre 0 et 160, la matière zéolithique isotactique cristalline possédant au moins les lignes de diffraction ci-après:

d(Å) = 11,1±0,3/10,0±0,25/3,85±0,1/
3,71±0,1/3,54±0,07,

à partir de mélanges de $YO_2$ et de $W_2O_3$ par une cristallisation hydrothermale à des températures de 100 à 200 °C, caractérisé en ce que la cristallisation est réalisée en présence d'amines organiques, en l'absence de bases de métaux alcalins, de métaux alcalino-terreux ou d'azote quaternaire et des précurseurs de celles-ci, le rapport molaire du $YO_2$ au $W_2O_3$ dans le mélange réactionnel étant réglé, en fonction de la longueur de chaîne $L_R$ de l'amine organique, pour obtenir la formation d'une zéolithe isotactique, les rapports molaires $H_2O$/amine étant compris entre 0,4 et 100 et les rapports molaires amine/$Al_2O_3$ entre 5 et 400.

2. Procédé de préparation de zéolithes alumino-silicatées suivant la revendication 1 à partir de mélanges de $SiO_2$ et de $Al(OH)_3$ par une cristallisation hydrothermale à des températures de 100 à 200 °C, caractérisé en ce que la cristallisation est réalisée en présence d'amines organiques, en l'absence de bases de métaux alcalins, de métaux alcalino-terreux ou d'azote quaternaire et des précurseurs de celles-ci, en établissant dans le mélange réactionnel, en fonction de la longueur de chaîne $L_R$ de l'amine organique, un rapport molaire

$$SiO_2/Al_2O_3 = (1 \pm 0.15) \frac{96 - \frac{40\ n}{L_R + 4}}{\frac{20\ n}{L_R + 4}}$$

les rapports molaires $H_2O$/amine étant compris entre 0,4 et 100 et les rapports molaires amine/$Al_2O_3$ entre 5 et 400.

3. Procédé de préparation de zéolithes boro-silicatées isotactiques cristallines suivant la revendication 1 à partir de mélanges de $SiO_2$ et de $B(OH)_3$ par une cristallisation hydrothermale à des températures de 100 à 200 °C, caractérisé en ce que la cristallisation est réalisée en présence d'amines organiques, en l'absence de bases de métaux alcalins, de métaux alcalino-terreux ou d'azote quaternaire et des précurseurs de celles-ci, la quantité de $B(OH)_3$ ajoutée au mélange réactionnel se limitant à celle nécessaire pour la formation de la zéolithe boro-silicatée isotactique cristalline.

4. Procédé suivant l'une des revendications 1 à 3, caractérisé en ce que la cristallisation hydrothermale est réalisée en présence de dipropylène-triamine.

5. Procédé suivant l'une des revendications 1 à 4, caractérisé en ce que la cristallisation hydrothermale est réalisée en présence de propylène-diamine.

6. Procédé suivant l'une des revendications 1 à 5, caractérisé en ce que la cristallisation hydrothermale est réalisée en présence de dihexaméthylène-triamine.

7. Procédé suivant l'une des revendications 1 à 6, caractérisé en ce que la cristallisation hydrothermale est réalisée en présence de triéthylènetétramine.

8. Procédé suivant l'une des revendications 1 à 7, caractérisé en ce que la cristallisation hydrothermale est réalisée en présence de diéthylènetriamine.

9. Procédé de préparation de zéolithes isotactiques cristallines suivant l'une des revendications 1 à 18 (lire 1 à 8), caractérisé en ce, par élimination de la composante aminée, on obtient les zéolithes dans leur forme H.

10. Zéolithes isotactiques cristallines de la composition

$$2/_nR \cdot W_2O_3 \cdot m\ YO_2 \cdot p\ H_2O,$$

dans laquelle R désigne la dipropylène-triamine, la dihexaméthylène-triamine, la triéthylène-tétramine, la propylène-diamine ou la diéthylène-triamine et n indique le nombre de groupes amine dans la molécule d'amine, W représente les éléments B, Al, Ga et Fe et Y les éléments Si et Ge et

$$m = (1 \pm 0.15)\ \frac{96 - \frac{40\ n}{L_R + K}}{\frac{20\ n}{L_R + K}}$$

où $L_R$ est la longueur de la molécule d'amine en Å et K est une constante d'une valeur d'environ 4 et p est un nombre entre 0 et 160, la matière zéolithique isotactique cristalline possédant au moins les lignes de diffraction ci-après:

d(Å) = 11,1 ± 0,3/10,0 ± 0,25/3,85 ± 0,1/ 3,71 ± 0,1/3,54 ± 0,07.

11. Utilisation de zéolithes suivant la revendication 10 comme catalyseurs pour la transformation d'alcools et (ou) d'éthers de dialkyle en oléfines et (ou) substances aromatiques.